# FASCICULE DE BREVET EUROPEEN

(11) **EP 0 843 814 B1**
(45) Date de publication et mention de la délivrance du brevet: **24.01.2001**
(21) Numéro de dépôt: 96928485.0
(22) Date de dépôt: 07.08.1996
(51) Int. Cl.: G01N 33/02, G01N 21/35

(54) **MESURE DE LA DIGESTIBILITE DES ALIMENTS CHEZ LES RUMINANTS**
MESSUNG DER FUTTERVERDAULICHKEIT BEI WIEDERKÄUERN
FEED DIGESTIBILITY MEASUREMENT IN RUMINANTS

(30) Priorité: 10.08.1995 FR 9509710
(43) Date de publication de la demande: 27.05.1998
(73) Titulaire: AVENTIS ANIMAL NUTRITION S.A., 92160 Antony (FR)
(72) Inventeur: WILLIAMS, Peter, F-78370 Plaisir (FR)
(74) Mandataire: Brachotte, Charles
(86) Numéro de dépôt international: FR9601255
(87) Numéro de publication internationale: WO9706434

(56) Documents cités:
- EP-A- 0 278 636
- DD-A- 254 594
- ANIMAL FEED SCIENCE AND TECHNOLOGY, vol. 54, Août 1995, ISSN 0377-8401, pages 203-216, XP000195541 ANTONIEWICZ A M ET AL: "RUMEN DEGRADABILITY OF CRUDE PROTEIN OF DRIED GRASS AND LUCERNE FORAGE MEASURED BY IN SACCO INCUBATION AND PREDICTED BY NEAR INFRARED SPECTROSCOPY"
- ANNALES DE ZOOTECHNIE, vol. 42, no. 3, Mars 1993, ISSN 0003-424X, pages 251-270, XP002003084 DARDENNE P ET AL: "COMPOSITION AND NUTRITIVE VALUE OF WHOLE MAIZE PLANTS FED FRESH TO SHEEP. II. PREDICTION OF THE IN VIVO ORGANIC MATTER DIGESTIBILITY" cité dans la demande
- JOURNAL OF CHEMOMETRICS, vol. 3, no. 2, Mars 1989, ISSN 0886-9383, pages 397-407, XP000008744 DOWNEY G ET AL: "DRIED GRASS SILAGE ANALYSIS BY NIR REFLECTANCE SPECTROSCOPY-A COMPARISON OF STEPWISE MULTIPLE LINEAR AND PRINCIPAL COMPONENT TECHNIQUES FOR CALIBRATION DEVELOPMENT ON RAW AND TRANSFORMED SPECTRAL DATA"

## Description

La présente invention concerne un procédé de détermination de la valeur nutritionelle d'ensilages donnés à des ruminants. Depuis longtemps on cherche à optimiser la valeur nutritive des aliments donnés aux animaux d'élevage afin de favoriser, en particulier pour les ruminants, la production laitière et/ou la production de viande.

Il est connu par exemple selon les publications parues dans Can. J. Anim. Sci 67; 557-562 (June 1987) qu'il est possible de déterminer aussi bien la digestibilité "in vitro" de divers hybrides de maïs que la même digestibilité par une méthode de détermination par spectrophotométrie dans le proche infrarouge.

Il est aussi connu selon l'article des auteurs suivants: Valdes, Hunter et Pinter paru dans Can. J. Plant Sci. 67; 747-754 (July 1987) de déterminer les valeurs IVMD (digestibilité de la matière sèche in vitro) qui était jusqu'alors réalisée par une technique de fermentation ruminale "in vitro", décrite par Tilley et Terry en 1963, par une méthode d'analyse spectrophotométrique dans le proche infrarouge.

Les méthodes décrites dans les deux publications précédentes ont permis d'associer des méthodes d'analyse de la digestibilité "in vitro" avec des méthodes d'analyse spectophotométrique dans le proche infrarouge. Cette association pour estimer la digestibilité "in vivo" de méthodes d'analyse chimique et enzymatique ayant elles même des variabilités importantes avec une méthode spectrophotométrique dans le proche infrarouge ayant elle aussi une variabilité importante permettait d'obtenir des appréciations de la digestibilité assez moyennes.

En ce qui concerne la prédiction de la digestibilité "in vivo" par analyse spectrophotométrique dans le proche infrarouge aucune publication n'est parue à ce jour. D'autres publications associant des analyses "in vitro" avec la spectrophotométrie dans le proche infrarouge sont parues telles que la publication de Dardenne, Andrieu, Barrière, Biston, Demarquilly, Femenias, Lila, Maupetit, Rivière et Ronsin, Ann. Zootech (1993) 42, 251-270 qui a établi des courbes de calibration pour les analyses suivantes matière sèche, cendres, protéines totales, les carbohydrates solubles dans l'eau, les celluloses, les constituants des parois cellulaires, les fibres totales, les fibres détergentes acides et neutres, la lignine, le solubilités enzymatiques déterminées par les méthodes de Aufrère, Limagrain et Lila. Ces différentes analyses "in vitro" permettent de déterminer comme précédemment une valeur de digestibilité "in vivo". L'ensemble de ces analyses "in vitro" a pu être réalisé grace à une détermination par spectrophotométrie dans le proche infrarouge ce qui a permis par calcul de déterminer une valeur de digestibilité "in vivo".

Aucune de ces méthodes n'a jamais à ce jour associé une détermination "in vivo" de la valeur nutritionelle d'un ensilage directement avec une analyse par spectrophotométrie dans le proche infrarouge. Cette détermination n'était pas évidente à la lecture de tout cet art antérieur essentiellement chimique et mathématique car dans l'organisme de l'animal apparaissent de nombreux phénomènes physiologiques qui n'ont jamais été pris en compte dans les analyses chimiques ou enzymatiques réalisées à ce jour et qui donc n'ont pu donner lieu à une détermination par spectrophotométrie dans le proche infrarouge.

La présente invention a pour objectif la détermination de la valeur nutritionelle "in vivo" d'aliments chez les ruminants par spectrophotométrie dans le proche infrarouge. Elle concerne plus particulièrement la détermination de la valeur de dégradabilité ruminale "in vivo" des fourrages chez les ruminants. Elle peut aussi concerner la valeur de digestibilité intestinale "in vivo" des acides aminés. Ainsi cette méthode peut permettre de déterminer la valeur de dégradabilité réelle des aliments au cours de leur transit digestif.

Parmi les aliments dont la valeur nutritionelle et la valeur de dégradabilité ruminale peuvent être déterminées par analyse dans le proche infrarouge on peut citer les ensilages et tout particulièrement les ensilages d'herbes, de maïs, de luzerne, de drèches, de pulpes de betteraves. Ces ensilages sont de préférence stockés dans de minisilos contenant environ 4kg d'ensilage, le prélèvement au niveau de ces minisilos est plus représentatif car plus homogène que dans des silos classiques.

Selon un plan pratique la méthode de détermination de la digestibilité ruminale consiste à inclure la matière première dont on désire déterminer la digestibilité dans des sachets de nylon, à inclure ces sachets de nylon dans le rumen de vaches canulées au niveau du rumen, à laisser les sachets un temps variable dans le rumen, les vaches étant alimentées normalement. Après le délai choisi les sachets sont sortis, lavés à l'eau et leurs contenus sont analysés.

Les analyses chimiques suivantes sont réalisées sur l'ensilage avant introduction dans le rumen :
- matières minérales
- matière azotée totale
- amidon
- cellulose brute
- NDF (fibres détergentes neutres)
- ADF(fibres détergentes acides)
- lignine

Après la période d'incubation dans le rumen les analyses suivantes sont réalisées :
- matière azotée totale (MAT)
- amidon
- matière sèche (MS)
- NDF (fibres détergentes neutres)

Les dégradabilités ruminales de l'azote, de l'amidon, de la matière sèche (DMS) et des fibres détergentes neutres sont déterminées par différence.

En ce qui concerne la digestibilité intestinale des matières alimentaires et plus particulièrement des aminoacides on procède comme suit. On analyse sur les sachets sortis du rumen la teneur en aminoacides. On introduit les sachets dans une solution de pepsine et d'acide à pH2 pendant une période de 2,5 heures. On introduit ensuite les sachets dans une vache par une canule duodénale lors du repas du soir, les sachets sont collectés dans les fécès le lendemain, lavés à l'eau puis séchés. A chacune des étapes on mesure la quantité d'aminoacides résiduelle et on détermine la quantité d'aminoacides disponible pour l'animal par différence.

Après obtention des résultats chimiques et "in sacco", chaque échantillon d'ensilage sous sa forme broyée et séchée est enregistré par l'intermédiaire d'un spectrophotomètre travaillant dans le proche infra-rouge (c'est àdire entre 700 et 2500nm). La mesure effectuée se présente sous la forme d'un spectre qui est en fait une information physico-chimique de l'échantillon.

L'étape suivante consiste à associer les données N.I.R. (données spectrales) avec les résultats déterminés par les analyses de valeur nutritionelle classiques décrites précédemment. Ce processus aboutit à des équations de prédictions permettant de déterminer directement en fonction du spectre N.I.R. la valeur nutritionnelle d'un ensilage. La gestion de ces opérations se fait par l'intermédiaire d'un système informatique commercial, vendu sous la dénomination "NIRS 2, version 3.00" et distribué par Infrasolt International I.S.I.

Ces opérations permettent par une méthode simple, peu coûteuse et non destructive de la matière première, de déterminer la valeur nutritive de différents types de fourrages en un temps très court sans utiliser de calculs mathématiques. Cette méthode peut être utilisée de manière simple par chaque éleveur désirant adapter au mieux la ration alimentaire qu'il va donner à ses animaux.

Les exemples suivants permettent de décrire l'invention de manière plus complète, ils ne doivent pas être considérés comme limitatifs de l'invention.

### EXEMPLES

### I - Animaux

### I-1. Animaux

6 vaches laitières taries, non gestantes, de poids similaire, canulées au niveau du rumen et du duodénum. L'expérience peut également être réalisée sur 3 vaches, chaque échantillon sera passé deux fois sur les 3 vaches.

### 1-2. Rationnement

La ration se compose de fourrage et de concentré : 7 kg de foin, 2 kg de concentré. Une pierre à lécher est mise à disposition des animaux pour l'apport en minéraux et en oligo-éléments.

La quantité de foin donnée est réajustée selon l'état de l'animal.

**TABLEAU 1:**

| **Composition du concentré** | |
|---|---|
| MATIERES PREMIERES | % |
| Orge | 41 |
| Pulpe de betterave deshydratée | 37 |
| Tourteau de soja 48 | 15 |
| Urée | 2 |
| Mélasse | 5 |

Valeurs au kg : 0,9 UFL - 125 PDIN - 100 PDIE - 3 g de P - 13 g de Ca.

La distribution de concentré et de fourrage se fait à 8 h et 17 h chaque jour.

Les animaux seront adaptés au régime 3 semaines avec le début de l'expérience.

**Il - Réception et traitement des échantillons**

**II - 1. Réception des échantillons**

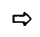 **Ouverture des mini-silos, enregistrement du poids et du pH**

Les ensilages de maïs sont stockés dans les mini-silos (environ 4 kg d'ensilage de maïs par mini-silo). Ces mini-silos sont ouverts environ 2 mois après leur confection. Le contenu de chaque mini-silo est versé dans un grand bac puis le bac est pesé et la quantité d'ensilage de chaque mini-silo est enregistrée. Le pH de l'ensilage est mesuré à l'aide d'un pHmètre en différents points de ce bac (au moins 5 mesures par bac) la moyenne des différentes mesures est ensuite réalisée.

**II - 2. Traitement des échantillons**

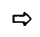 **Détermination de la matière sèche et séchage de l'échantillon**

Cinquante grammes d'ensilage sont prélevés dans le bac placé à l'étuve à 104°C pendant 16 h pour détermination de la matière sèche. Le poids de l'échantillon est enregistré au bout de 16 h puis 2 h plus tard. Ces mesures permettent d'obtenir 2 valeurs de matière sèche, la moyenne de ces deux valeurs est réalisée.

Le reste de l'ensilage contenu dans le bac est placé dans une autre étuve à 60°C pendant environ 3 jours. Ce délai varie selon la quantité d'ensilage à sécher. Le bac est retiré de l'étuve lorsque l'ensilage est parfaitement sec.

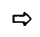 **Broyage de l'échantillon**

L'ensemble de l'échantillon est broyé avec un broyeur à couteau avec un tamis de 3 mm.

L'ensilage broyé est séparé en 3 échantillons : un échantillon (200 g) est envoyé pour analyse chimique, un autre échantillon (150 g) est utilisé pour les cinétiques de dégradation et le reste de l'échantillon est placé dans une boîte et est conservé en chambre froide à 4°C.

### III - Méthodes in sacco

La technique des sachets Nylon est réalisée à partir de la méthode INRA (Michalet - Doreau 1987).

**Ill - 1. Préparation des sachets**

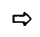 **Caractéristiques des sachets**

- Dimensions internes : 6 - 11 cm
- Porosité : 48 µm
- Quantité d'échantillon : 4 g
- Ratio poids/surface : 30 mg/cm²
- identification des sachets : N° d'échantillon, vache (A, B, C, D, E, F), N° de cinétique (de 1 à 6)

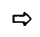 **Mode opératoire**

Le sachets vides sont laissés à l'étuve pendant 24 h à 80°C. Il sont ensuite pesés puis remplis avec 4 g d'échantillon et soudés. Il sont pesés remplis et fixés à une chaîne lestée : 36 sachets par chaînes.

### III - 2. Incubation ruminale

Chaque échantillon d'ensilage est incubé sur les 6 vaches. Les résidus de l'incubation seront ensuite regroupés par point de cinétique pour l'analyse.

**TABLEAU 2:**

| **Chronologie de chaque cinétique** | | |
|---|---|---|
| 1er Jour | 2ème jour | 3ème Jour |
| 7 h 45 : Introduction des sachets 2, 4, 8, 24 et 48 h | 7 h 45 : Sortie des sachets 16 h, 24 h | 7 h 45 : Sortie des sachets 48 h |
| 8 h : Repas | 8 h : Repas | 8 h : Repas |
| 9 h 45 : Sortie des sachets 2 h | | |
| 11 h 45 : Sortie des sachets 4 h | | |
| 15 h 45 : Sortie des sachets 8 h, introduction des sachets 16 h | | |
| 17 h : repas | 17 h : Repas | 17 h : Repas |

### III - 3. Lavage des sachets

Les sachets sont lavés à la sortie du rumen à l'eau froide dans une machine à laver de type Calor par cycle de 5 mn jusqu'à obtention d'une eau de rinçage claire. Les sachets sont ensuite placés au congélateur jusqu'à la fin de l'incubation ruminale puis à l'étuve pendant 16 h à 104°C. Ils sont pesés au bout de 16 h puis 2 h plus tard. La plus faible des 2 valeurs est conservée pour les calculs ultérieurs.

Les résidus de cinétique sont ensuite regroupés par point de cinétique pour être envoyés à l'analyse.

### IV - Analyses

### IV - 1. Analyse chimique sur le produit brut

Deux cent grammes d'ensilage de maïs séché et broyé est envoyé à l'analyse pour dosage des MM, MAT, amidon, CB, NDF, ADF, lignine. Les valeurs UFL et UFV sont calculées.

### IV - 2. Analyse chimique sur les résidus de cinétique

Trois dosages sont réalisés sur les résidus d'incubation, ils sont présentés dans le tableau 3.

**TABLEAU 3:**

| **Dosages réalisés sur les résidus** | |
|---|---|
| Points de cinétiques | Dosages |
| 2 h | MAT, amidon |
| 4 h | MAT, amidon |
| 8 h | MAT, amidon |
| 16 h | MAT, CB |
| 24 h | MAT, CB |
| 48 h | MAT |

### IV - 3. Calcul des valeurs PDI

Les valeurs PDIA, PDIE, PDIN sont calculées à l'issue des analyses chimiques et des cinétiques de dégradabilité en tenant compte des teneurs en CB, MAT et de la DT réelle.

**V - Analyse par le proche infra-rouge (N.I.R.)**

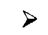 Traitement des échantillons

L'analyse infra-rouge est effectuée sur des échantillons ayant été préparés selon la procédure décrite au paragraphe 11-2, c'est à dire après séchage et broyage. La quantité de matière première utilisée pour ce type de mesure est de l'ordre de 150 à 200 grammes en fonction de la densité du produit.

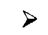 Enregistrement des spectres N.I.R.

Cette opération s'effectue par l'intermédiaire d'un spectrophotomètre à réseau de type N.I.R.S. 6500 (distribué par Perstorp Analytical Int.) fonctionnant selon le mode "Réflectance". L'une des principales spécificités de cet instrument est de travailler sur l'ensemble du proche infra-rouge et non pas sur quelques longueurs d'ondes spécifiques, comme cela est le cas pour certains appareils plus classiques.

L'échantillon est conditionné dans une cellule de transport rectangulaire se composant de trois faces opaques et d'une face munie d'un quartz. Après positionnement de la cellule dans le spectrophotomètre, la lumière réfléchie par l'échantillon qui est porteuse d'informations sur la composition chimique de ce dernier va être enregistrée sous forme d'un spectre. Chaque type de matière première et par conséquence chaque échantillon possède un spectre différent.

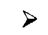 Constrution d'une population de calibration

Cette opération consiste à élaborer à partir de l'ensemble des échantillons dont on possède l'identité spectrale et les résultats chimiques et "in sacco" (MAT, NDF, ADF, DMS, DT) une base de données représentative des ensilages étudiés. Au sein d'un même fichier de calibration, il y a donc mise en commun des données numériques et spectrales.

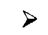 Centrage de la population de calibration

L'ensemble du fichier de calibration construit est soumis à un calcul statistique qui permet de détecter la présence de spectres hors normes qui présentent des distances de Mahalabonis (distance H statistique) trop importantes par rapport au spectre moyen de la population (centre de gravité). Une fois détectée, ces échantillons sont éliminés afin d'obtenir une population homogène.

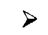 Calibration

Le fichier de calibration contenant l'ensemble des échantillons est soumis à un traitement statistique, dont il existe différents types : "Modified Partial Least Square" (M.P.L.S.), Partial Least Square (P.L.S.) qui est une forme générale de régression par composantes principales ou encore "Step Up" qui correspond à une régression linéaire plus simple. Après avoir choisi la méthode de régression adéquate, il est préférable d'effectuer un traitement mathématique par dérivation pour toutes les analyses. Ce traitement mathématique est déterminé par une séquence d'évaluation a, b, c, d: a=ordre de la dérivée, b=intervalle en nm sur lequel porte le calcul de la dérivée, c=constante 1 de lissage, d=constante 2 de lissage. Après initialisation par les choix précédents, une phase de calibration est lancée.

Une courbe de calibration est jointe en annexe.

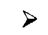 Utilisation des équations de calibration

Les déterminations par lecture infra-rouge sur des échantillons d'ensilages inconnus, sont réalisées à partir des équations de prédictions les plus performantes. Cette sélection s'opère en fonction de différents paramètres statistiques tels que le coefficient de corrélation (R²) ou encore l'erreur standard de prédiction.

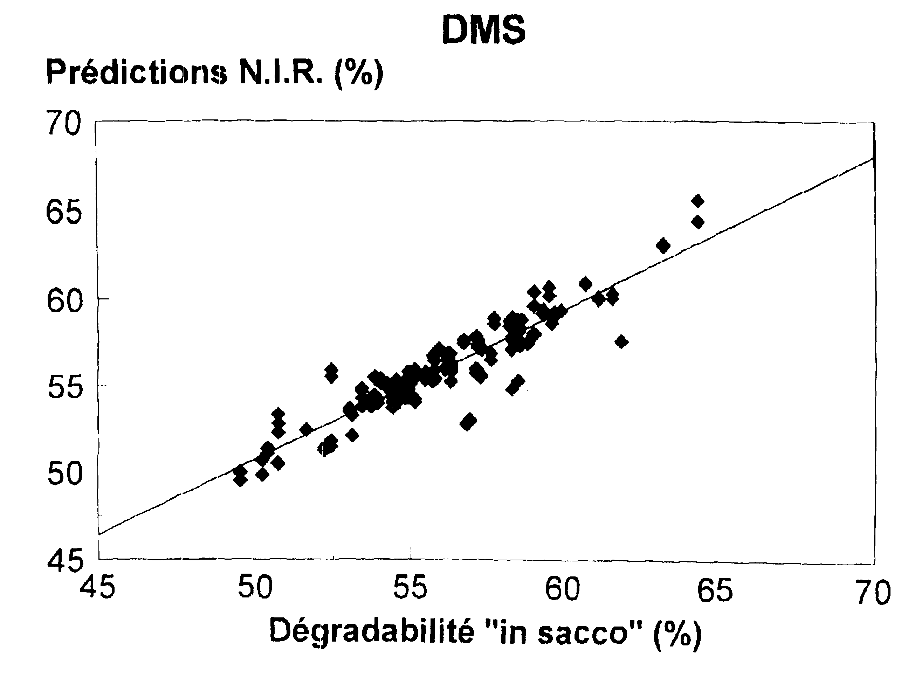

## Revendications

1. Procédé de détermination directe de la valeur nutritionelle "in vivo", lors du transit digestif d'ensilages chez les ruminants caractérisé en ce que l'on analyse la valeur nutritionelle desdits ensilages par spectrophotométrie dans le proche infrarouge.

2. Procédé de détermination de la valeur de dégradabilité ruminale "in vivo" des ensilages chez les ruminants caractérisé en ce que cette valeur est déterminée par spectrophotométrie dans le proche infrarouge.

3. Procédé de détermination de la valeur de digestibilité intestinale "in vivo" des acides aminés des ensilages chez les ruminants caractérisé en ce que cette valeur est déterminée par spectrophotométrie dans le proche infrarouge.

4. Procédé selon l'une quelconque des revendications précédentes caractérisé en ce que les ensilages sont choisis parmi les ensilages d'herbes, de maïs, de luzerne, de drèches, de pulpes de betteraves .

5. Procédé de détermination de la valeur alimentaire d'ensilages caractérisé en que ces ensilages sont stockés dans de minisilos contenant environ 4kg d'ensilage, des échantillons bruts (non digérés) sont prélevés et inclus dans des sachets qui sont introduits dans le rumen de l'animal pendant le temps nécessaire à la digestion desdits ensilages, puis à l'issue de la digestion, leur contenu est analysé par voie chimique ou enzymatique, et les données analytiques obtenues sont utilisées pour tracer une courbe de correlation mettant en parallèle pour le même ensilage les valeurs résultant de l'analyse spectrale dans le proche infrarouge d'échantillons bruts et les valeurs d'analyse chimique ou enzymatique d'échantillons après digestion dans le rumen.

## Patentansprüche

1. Verfahren zur direkten Bestimmung des in vivo-Nährwerts von Silagen im Verdauungsgang bei Wiederkäuern, dadurch gekennzeichnet, daß der Nährwert der Silagen spektrophotometrisch im nahen IR-Bereich analysiert wird.

2. Verfahren zur Bestimmung des Werts der ruminalen in vivo-Abbaubarkeit von Silagen bei Wiederkäuern, dadurch gekennzeichnet, daß dieser Wert spektrophotometrisch im nahen IR-Bereich bestimmt wird.

3. Verfahren zur Bestimmung der intestinalen in vivo-Verdaubarkeit von Aminosäuren von Silagen bei Wiederkäuern, dadurch gekennzeichnet, daß dieser Wert spektrophotometrisch im nahen IR-Bereich bestimmt wird.

4. Verfahren nach einem der vorstehenden Ansprüche, dadurch gekennzeichnet, daß die Silagen unter Silagen von Gras, Mais, Luzerne, Trebern und Rübenpulpe ausgewählt sind.

5. Verfahren zur Bestimmung des Nährwerts von Silagen, dadurch gekennzeichnet, daß die Silagen in Minisilos mit einem Gehalt an etwa 4 kg Silage gelagert werden, die rohen (nicht verdauten) Proben entnommen und in Beutel gefüllt werden, die dann in das Rumen des Tiers für die zur Verdauung dieser Silagen erforderliche Zeitspanne eingeführt werden, anschließend am Ende der Verdauung ihr Inhalt auf chemischem oder enzymatischem Wege analysiert wird und die erhaltenen analytischen Daten zur Aufstellung einer Korrelationskurve verwendet werden, auf der für die gleiche Silage die Werte der Spektralanalyse im nahen IR-Bereich für die rohen Proben und die Werte der chemischen oder enzymatischen Analyse der Proben nach der Verdauung im Rumen gegenübergestellt werden.

## Claims

1. Process for determining directly the "in vivo" nutritional value, during transit of silage through the digestive system of ruminants, characterized in that the nutritional value of the said silage is analysed by near-infra-red spectrophotometry.

2. Process for determining the "in vivo" ruminal degradability value of silage in ruminants, characterized in that this value is determined by near-infra-red spectrophotometry.

3. Process for determining the "in vivo" intestinal digestibility value of amino acids in silage in ruminants, characterized in that this value is determined by near-infra-red spectrophotometry.

4. Process according to any one of the preceding claims, characterized in that the silage is chosen from grass silage, corn silage, alfalfa silage, spent grain silage and beetroot pulp silage.

5. Process for determining the dietary value of silage, characterized in that this silage is stored in minisilos containing about 4 kg of silage, crude (undigested) samples are taken and placed in sachets which are introduced into the animal's rumen for a period of time necessary for digestion of the said silage, and then, after digestion, their contents are analysed chemically or enzymatically, and the analytical data obtained are used to plot a correlation curve which places in parallel, for the same silage, the values resulting from the near-infra-red spectral analysis of crude samples and the chemical or enzymatic analytical values of samples after digestion in the rumen.
